# EUROPÄISCHE PATENTANMELDUNG

(11) **EP 0 543 226 A2**
(43) Veröffentlichungstag der Anmeldung: **26.05.1993**
(21) Anmeldenummer: 92118949.4
(22) Anmeldetag: 05.11.1992
(51) Int. Cl.: C07C 47/04, C07C 45/80, C07C 45/85, C07C 45/51, C07C 45/82, C07C 45/83

(54) **Verfahren zur Herstellung von wasserfreiem un von Verunreinigungen befreitem Formaldehyd**

(30) Priorität: 16.11.1991 DE 4137846
(71) Anmelder: BASF Aktiengesellschaft, D-67063 Ludwigshafen (DE)
(72) Erfinder: Knuth, Bernhard, Dr., W-6711 Laumersheim (DE); Vogel, Herbert, Dr., W-6700 Ludwigshafen (DE); Harder, Wolfgang, Dr., W-6940 Weinheim (DE); Gehrer, Eugen, Dr., W-6700 Ludwigshafen (DE)

(57) **Zusammenfassung**

Verfahren zur Herstellung von weitgehend wasserfreiem und weitgehend von Verunreinigungen befreitem Formaldehyd aus wäßrigen Formaldehydlösungen, dadurch gekennzeichnet, daß die wäßrige Formaldehydlösung mit einem Alkohol extrahiert, die erhaltene alkoholische Halbacetallösung von Wasser befreit und danach das Halbacetal in einer nachfolgenden Lösungsmittelwechselkolonne in Gegenwart eines Lösungsmittels mit einem tieferen Siedepunkt als der bei der Extraktion verwendete Alkohol, thermisch gespalten und als Kopfprodukt Formaldehyd im Gemisch mit dem Lösungsmittel abgezogen wird.

## Beschreibung

Die vorliegende Erfindung betrifft ein verbessertes Verfahren zur Herstellung von weitgehend wasserfreiem und weitgehend von Verunreinigungen befreitem Formaldehyd.

Viele Folgeprodukte des Formaldehyds können nur in Abwesenheit von Wasser hergestellt werden. Beispielsweise genannt sei die Herstellung von Dihydroxyaceton. Aus Methanol hergestellter Formaldehyd enthält jedoch immer Wasser und muß daher durch ein geeignetes Verfahren entwässert werden.

Bei bekannten Verfahren zur Herstellung von wasserfreiem Formaldehyd wird Formaldehyd mit einem Alkohol zum Halbacetal umgesetzt und anschließend durch thermische Spaltung des Halbacetals zurückgewonnen. Ein solches Verfahren ist z.B. in der Belgischen Patentschrift BE-A-722 900 beschrieben. Nachteilig bei diesem Verfahren ist, daß der erhaltene Formaldehyd durch beträchtliche Anteile an Wasser und Alkohol verunreinigt ist, so daß eine weitere Aufarbeitung notwendig wird.

Beim Verfahren der Deutschen Patentschrift DE-B-1 057 086 wird die Lösung des Formaldehydhalbacetals vor der thermischen Spaltung entwässert. Auch der bei diesem Verfahren erhaltene Formaldehyd muß weiter aufgearbeitet werden, um eine ausreichende Reinheit und Wasserfreiheit zu erzielen. Insbesondere kommt es bei der Reinigung des Formaldehyds durch Kondensationsschritte vielfältig zu Ablagerungen von polymerisiertem Formaldehyd in den Apparaturen. Um solche Ablagerungen zu vermeiden wird nach dem Verfahren der JP-A-53 040 707 zu den Pyrolyseprodukten der Halbacetalspaltung zusätzlich ein Inertgas zugesetzt. Aus der erhaltenen Gasmischung wird der Alkohol durch eine Kondensation bei tiefen Temperaturen abgetrennt.

Nach dem Verfahren der DE-A-1 090 191 erfolgt die Aufarbeitung der bei der Pyrolyse des Formaldehydhalbacetals entstehenden Produkte durch eine Gegenstromwäsche mit der Formaldehydhalbacetallösung selbst. Ein Lösungsmittelaustausch ist nicht erwähnt.

Allen bisher bekannten Verfahren zur Herstellung von wasserfreiem Formaldehyd ist gemeinsam, daß eine aufwendige Aufarbeitung des bei der Pyrolyse des Formaldehydhalbacetals erhaltenen Formaldehyds notwendig ist, um die gewünschte Wasserfreiheit zu erreichen. Die Trennung des Formaldehyd/Alkohol-Gemisches erfolgt dabei im allgemeinen durch Kondensationsschritte. Insbesondere protische Verbindungen wie Wasser, Säuren, z.B. Ameisensäure und der für die Halbacetalbildung eingesetzte Alkohol verbleiben dabei vielfach als Verunreinigungen im Formaldehyd.

Aufgabe der Erfindung war daher ein Verfahren zur Herstellung von weitgehend wasserfreiem und weitgehend von Verunreinigungen befreitem Formaldehyd, bei dem auf eine aufwendig Aufarbeitung, insbesondere eine technisch aufwendige Teilkondensation der bei der Pyrolyse des Formaldehydhalbacetals erhaltenen alkoholischen Formaldehydlösung, verzichtet werden kann.

Demgemäß wurde ein Verfahren zur Herstellung von weitgehend wasserfreiem und weitgehend von Verunreinigungen befreitem Formaldehyd gefunden, das dadurch gekennzeichnet ist, daß die wäßrige Formaldehydlösung mit einem Alkohol extrahiert, die erhaltene alkoholische Halbacetallösung von Wasser befreit und danach das Halbacetal in einer nachfolgenden Lösungsmittelwechselkolonne in Gegenwart eines Lösungsmittels mit einem tieferen Siedepunkt als der bei der Extraktion verwendete Alkohol, thermisch gespalten und als Kopfprodukt Formaldehyd im Gemisch mit dem Lösungsmittel abdestilliert wird.

Bevorzugte Ausführungen des Verfahrens sind den Unteransprüchen zu entnehmen.

Durch das erfindungsgemäße Verfahren ist ein weitgehend von Wasser und sonstigen Verunreinigungen, insbesondere protischen Verbindungen, wie Alkoholen oder Ameisensäure befreites Formaldehyd erhältlich. Eine aufwendige Aufarbeitung der bei der Pyrolyse des Formaldehydhalbacetals erhaltenen Formaldehyds entfällt. Der erhaltene Formaldehyd kann als Gemisch mit einem nicht alkoholischen, gegenüber Formaldehyd inerten Lösungsmittel erhalten werden und kann in oder aus diesem Gemisch direkt zu Folgeprodukten verarbeitet werden.

Formaldehyd wird technisch im allgemeinen durch Oxidation von Methanol hergestellt. Dabei werden üblicherweise wäßrige Formaldehydlösungen erhalten. Der Formaldehydgehalt dieser Lösungen kann nahezu beliebig eingestellt werden, üblich vor allem sind jedoch Konzentrationen zwischen 30 und 60 Gew.-% Formaldehyd.

Der Formaldehyd wird im ersten Verfahrensschritt mit einem Alkohol zum Halbacetal umgesetzt. Insbesondere eignen sich als Alkohole C₄-C₁₂-Alkanole, z.B. Octanol, iso-Octanole, wie 2-Ethylhexanol, Cyclohexanol oder Benzylalkohol. Zur Bildung des Halbacetals kann der Alkohol durch bekannte Extraktionsverfahren mit der wäßrigen Formaldehydlösung in Kontakt gebracht werden. Als besonders empfehlenswert hat sich eine mehrstufige Extraktion, z.B. in einer Mixer-Settler Apparatur erwiesen.

Bei der Extraktion in einer Mixer-Settler Apparatur werden die organische Phase (der Alkohol) und die wäßrige Formaldehydphase in Mischgefäßen unter Rühren vermischt, und anschließend in ein Gefäß ohne Rührung (Settler) zur Auftrennung der Phasen gegeben.

Die Halbacetalbildung verläuft schon bei Raumtemperatur und Normaldruck mit ausreichender Geschwindigkeit und bei Einhaltung ausreichender Kontaktzeiten quantitativ; die Kontaktzeit sollte insbesondere größer als 5 Minuten sein; längere Kontaktzeiten als 2 Stunden bewirken im allgemeinen keine weitere Verbesserung des Extraktionsergebnisses mehr.

Bei einer mehrstufigen Extraktion in einer Mixer/Settler Apparatur empfiehlt es sich, eine Mindestkontaktzeit von 5 Minuten im Mixer in jeder Stufe einzuhalten. Sehr gute Ergebnisse werden mit einer 2 bis 6, insbesondere 3 bis 5 stufigen Extraktion erzielt.

Die Extraktionstemperatur beträgt bevorzugt 20 bis 100°C besonders bevorzugt 50 bis 80°C. Bei zu hohen Temperaturen wird der Verteilungskoeffizient (Formaldehyd-Konzentrationen in der organischen Alkoholphase/Formaldehyd-Konzentration in der wäßrigen Phase) ungünstiger, bei zu niedrigen Temperaturen muß die Kontaktzeit zwischen organischer Alkoholphase und wäßriger Phase in den Mixern zu stark angehoben werden, was zu unwirtschaftlich großen Mixerbehältern führt.

Bei 20 bis 100°C und mehrstufiger Verfahrensweise haben sich in den Mixern Kontaktzeiten von jeweils 15 bis 30 Minuten als besonders geeignet erwiesen.

Nach der Extraktion wird eine alkoholische Lösung erhalten, welche das Formaldehydhalbacetal, gegebenenfalls nicht umgesetztes Formaldehyd und Wasser enthält. Die in der Extraktionsstufe abgetrennte wäßrige Phase enthält nahezu kein Formaldehyd mehr und kann, falls gewünscht, weiter aufgearbeitet und für andere Zwecke verwendet werden.

Die alkoholische Lösung wird in der folgenden Verfahrensstufe weitgehend von Wasser befreit.

Die Lösung kann dazu in eine sog. Entwässerungskolonne gegeben werden. Diese Kolonne wird vorzugsweise im Vakuum, insbesondere bei 10 bis 100 mbar betrieben. Auf einen Einsatz eines Schleppmittels für das Wasser (z.B. Azeotropbildner) kann so verzichtet werden. Bei Vakuum kann weiterhin die Sumpftemperatur so niedrig gehalten werden, daß nahezu noch keine Zersetzung des Halbacetals in Formaldehyd und Alkohol zu beobachten ist. Die Sumpftemperatur wird bevorzugt so eingestellt, daß die alkoholische Lösung siedet. Im allgemeinen werden die Temperaturen zwischen 50 und 150, insbesondere 75 und 120°C liegen.

Die Temperatur am Kolonnenkopf liegt geeigneterweise bei der Kondensationstemperatur der dort vorliegenden Wasser/Alkohol-Gemische. Im allgemeinen ergeben sich Temperaturen zwischen 5 und 75, insbesondere 5 und 50°C.

Bei der Entwässerungskolonne kann es sich um Kolonnen verschiedener Bauart handeln. In Betracht kommen z.B. sowohl Füllkörperkolonnen als auch Bodenkolonnen mit Glockenböden, Siebböden oder ähnlichem. Die Trennleistung ist im wesentlichen abhängig von der Zahl der theoretischen Böden. Im allgemeinen sind 10 bis 20 theoretische Böden ausreichend.

Das am Kolonnenkopf anfallende Wasser/Alkohol-Gemisch wird nach der Kondensation bevorzugt in zwei Phasen getrennt. Die obere Alkoholphase kann als RÜcklauf auf die Entwässerungskolonne gegeben werden. Die untere wäßrige Phase, die noch gelösten Alkohol und geringe Mengen an Formaldehyd enthält, wird aus wirtschaftlichen Gründen bevorzugt in die Extraktionsstufe zurückgeführt (s. Zeichnung).

Aus der Lösung im Kolonnensumpf abdestilliertes Formaldehyd, welches am Kolonnenkopf bei den gegebenen Temperaturen noch gasförmig ist, kann durch Einleiten in Alkohol absorbiert (Alkoholquench) und die erhaltene Lösung als Rücklauf auf die Kolonne gegeben werden. So wird weitgehend vermieden, daß das in untergeordneten Mengen vorhandene freie Formaldehyd die Vakuumleitungen und apparativen Einrichtungen belegt.

Im Sumpf der Kolonne liegt eine alkoholische Lösung vor, welche das Formaldehydhalbacetal und deutlich verringerte Mengen Wasser enthält.

Das Sumpfprodukt wird in eine weitere Kolonne geleitet, in der das Formaldehydhalbacetal wieder in Formaldehyd und Alkohol gespalten und gleichzeitig ein Lösungsmittelaustausch vorgenommen wird (Lösungsmittelwechselkolonne). Vorzugsweise wird dazu die alkoholische Lösung aus der Entwässerungskolonne auf einen mittleren Boden der Lösungsmittelwechselkolonne gegeben, so daß sich die Kolonne in einen Auftriebs- und einen Abtriebsteil gliedert. Im allgemeinen kann mit 10 bis 20 theoretischen Böden sowohl im Auftriebs- als auch im Abtriebsteil eine gute Reinheit des Formaldehyds erzielt werden.

Die Kolonne wird geeigneterweise bei einem Druck zwischen 10 mbar und Normaldruck betrieben. Aus naheliegenden wirtschaftlichen Gründen ist Normaldruck bevorzugt. Als Sumpftemperatur wird bevorzugt die Siedetemperatur des Alkohols eingestellt. Insbesondere liegt die Temperatur zwischen 160 und 200°C. Im Sumpf der Kolonne wird das Formaldehydhalbacetal in Formaldehyd und Alkohol gespalten.

Der aus dem Sumpf abgezogene formaldehydfreie Alkohol kann sowohl in die Extraktionsstufe als auch über den Alkoholquench des Formaldehyds in die Entwässerungskolonne zurückgeführt werden.

Auf einen oberen Boden der Lösungsmittelwechselkolonne wird gleichzeitig ein Lösungsmittel eingeleitet, welches einen tieferen Siedepunkt als der eingesetzte Alkohol aufweist.

Die Kopftemperatur der Kolonne wird so gewählt, daß dieses Lösungsmittel und Formaldehyd gasförmig sind, so daß als Kopfprodukt der Lösungsmittelwechselkolonne ein Gasgemisch aus Formaldehyd und dem gewünschten Lösungsmittel abgezogen wird.

Als Lösungsmittel kommen insbesondere gegenüber Formaldehyd inerte Lösungsmittel in Betracht. Geeignet sind z.B. Dimethylformamid, Tetrahydrofuran, lineare und cyclische aliphatische Kohlenwasserstoffe wie Cyclohexan. Auch nicht gegenüber Formaldehyd inerte Lösungsmittel können Verwendung finden. Alkohol als Lösungsmittel sind z.B. dann notwendig, wenn das Formaldehyd wieder in ein Halbacetal, z.B. mit einem C₁-C₃-Alkohol, überführt werden soll.

Der Gehalt an Restwasser und anderen Verunreinigungen, z.B. Ameisensäure und Alkohol im erhaltenen Formaldehyd ist sehr gering. Wassergehalte von unter 0,05 % können erreicht werden. Der für die Bildung des Formaldehydhalbacetals eingesetzte Alkohol ist ohne aufwendige Kondensationsschritte abgetrennt.

Als Lösungsmittelwechselkolonne eignen sich ebenfalls Kolonnen wie z.B. die genannten Füllkörperkolonnen oder Bodenkolonnen.

Ein Vorteil des Verfahrens ist u.a. auch darin zu sehen, daß der weitgehend wasserfreie Formaldehyd direkt in ein Lösungsmittel überführt werden kann, welches für die weitere Verwendung bzw. weitere Umsetzung am geeignetesten ist.

So kann sich z.B. bei Verwendung eines gegenüber Formaldehyd inerten LÖsungsmittels in Gegenwart eines Thiazoliumsalzes direkt eine Umsetzung zu Dihydroxyaceton durchgeführt werden.

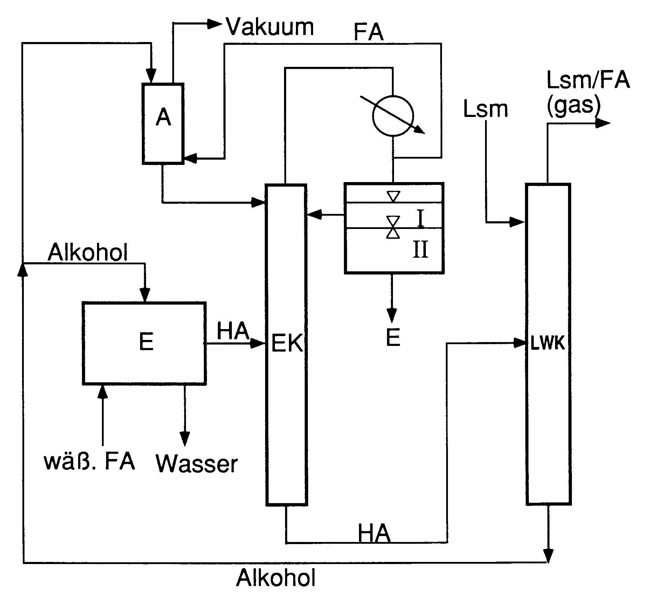

Die Zeichnung stellt einen bevorzugten Aufbau zur Durchführung des Verfahrens dar.

Alkohol und wäßrige Formaldehydlösung (wäß. FA) werden der Extraktionsstufe (E) zugeführt. Die erhaltene wäßrige Phase wird abgetrennt und die alkoholische Lösung des Formaldehydhalbacetals (HA) der Entwässerungskolonne (EK) zugeführt. Das als Kopfprodukt der Entwässerungskolonne anfallende Wasser/Alkohol-Gemisch bildet nach Absetzen eine obere alkoholische Phase I aus, welche als Rücklauf auf die Entwässerungskolonne gegeben, und eine untere wäßrige Phase II, welche in die Extraktion zurückgeführt wird. Gasförmiges Formaldehyd (FA) wird in einem Absorber in Alkohol absorbiert (Alkoholquensch). Die entwässerte Halbacetallösung wird in eine Lösungsmittelwechselkolonne (LWK) gegeben. Auf einen oberen Boden der Lösungsmittelwechselkolonne wird ein Lösungsmittel (Lsm) zudosiert, welches einen tieferen Siedepunkt als der Alkohol hat. Am Kopf der Kolonne wird ein gasförmiges Gemisch von Formaldehyd und dem Lösungsmittel abgezogen. Der im Kolonnensumpf anfallende Alkohol wird in die Extraktionsstufe zurückgeführt.

### Beispiel

### Verfahren zur Herstellung von wasserfreiem Formaldehyd

250 g/h einer 30 gew.%igen wäßrigen Formaldehydlösung wurden in einer vierstufigen Mixer-Settler-Kaskade im Gegenstrom mit 400 g/h 2-Ethylhexanol extrahiert (Zeichnung). Die erhaltene alkoholische Halbacetallösung wurde als Seitenstrom in die Entwässerungskolonne, die bei einem Kopfdruck von 40 mbar betrieben wurde, gegeben. Bei einer Sumpftemperatur von 97°C destillierten etwa 26 ml/h Wasser über Kopf. Der Sumpfaustrag wurde in die Mitte der Lösungsmittelwechselkolonne geleitet, in die am Kolonnenkopf 600 ml/h DMF aufgegeben wurde. Die Sumpf- und Kopftemperaturen betrugen 184°C bzw. 153°C. Die Formaldehyd/DMF-Brüden, die am Kopf der Lösungsmittelwechselkolonne abgezogen wurden, enthielten noch 250 ppm Wasser und 8 ppm Ameisensäure.

## Patentansprüche

1. Verfahren zur Herstellung von weitgehend wasserfreiem und weitgehend von Verunreinigungen befreitem Formaldehyd aus wäßrigen Formaldehydlösungen, dadurch gekennzeichnet, daß die wäßrige Formaldehydlösung mit einem Alkohol extrahiert, die erhaltene alkoholische Halbacetallösung von Wasser befreit und danach das Halbacetal in einer nachfolgenden Lösungsmittelwechselkolonne in Gegenwart eines Lösungsmittels mit einem tieferen Siedepunkt als der bei der Extraktion verwendete Alkohol, thermisch gespalten und als Kopfprodukt Formaldehyd im Gemisch mit dem Lösungsmittel abgezogen wird.

2. Verfahren nach Anspruch 1, dadurch gekennzeichnet, daß die Umsetzung der Formaldehyds zum Halbacetal in einer mehrstufigen Mixer-Settler-Apparatur erfolgt.

3. Verfahren nach Anspruch 1 oder 2, dadurch gekennzeichnet, daß die Entwässerung der Halbacetallösung in einer Vakuumkolonne durchgeführt wird.

4. Verfahren nach einem der Ansprüche 1 bis 3, dadurch gekennzeichnet, daß das bei der Entwässerung der Halbacetallösung erhaltene Wasser/Alkohol-Gemisch kondensiert wird und die dabei entstandene alkoholische Phase als Rücklauf in die Entwässerungskolonne sowie die wäßrige Phase in die Extraktionsstufe zurückgeführt wird.
